# EUROPEAN PATENT SPECIFICATION

(11) **EP 3 078 370 B1**
(45) Date of publication and mention of the grant of the patent: **07.11.2018**
(21) Application number: 16167881.8
(22) Date of filing: 14.09.2012
(51) Int. Cl.: A61K 9/48, A61K 9/28, A61K 9/50, A61K 31/485

(54) **TAMPER RESISTANT IMMEDIATE RELEASE FORMULATIONS**
MANIPULATIONSSICHERE FORMULIERUNGEN FÜR SOFORTIGE FREISETZUNG
FORMULATIONS À LIBÉRATION IMMÉDIATE INVIOLABLE

(30) Priority: 16.09.2011 US 201161535758 P
(43) Date of publication of application: 12.10.2016
(62) Divisional of application: 12772421.9
(73) Proprietor: Purdue Pharma L.P., Stamford, CT 06901 (US)
(72) Inventor: Reilly, Kevin, Kendall Park, NJ New Jersey 08824 (US)
(74) Representative: Maiwald Patent- und Rechtsanwaltsgesellschaft mbH

(56) References cited:
- EP-A1- 1 897 545
- WO-A2-2005/041934
- WO-A2-2009/023672

## Description

### FIELD OF THE INVENTION

The present invention relates to the field of pharmaceutical dosage forms that are resistant to tampering and abuse.

### BACKGROUND

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Opioid agonist formulations intended for oral use are sometimes crushed or subject to extraction with solvents (e.g., ethanol) by drug abusers to provide the opioid contained therein for non-prescribed illicit use (e.g., nasal or parenteral administration).

There have previously been attempts in the art to control the abuse potential associated with opioid analgesics. For example, the combination of pentazocine and naloxone has been utilized in tablets available in the United States, commercially available as Talwin® Nx from Sanofi-Winthrop. Talwin® Nx contains pentazocine hydrochloride equivalent to 50 mg base and naloxone hydrochloride equivalent to 0.5 mg base. Talwin® Nx is indicated for the relief of moderate to severe pain. The amount of naloxone present in this combination has low activity when taken orally, and minimally interferes with the pharmacologic action of pentazocine. However, this amount of naloxone given parenterally has profound antagonistic action to narcotic analgesics. Thus, the inclusion of naloxone is intended to curb a form of misuse of oral pentazocine which occurs when the dosage form is solubilized and injected. Therefore, this dosage has lower potential for parenteral misuse than previous oral pentazocine formulations. A fixed combination therapy comprising tilidine (50 mg) and naloxone (4 mg) has been available in Germany for the management of severe pain since 1978 (Valoron® N, Goedecke). The rationale for the combination of these drugs is effective pain relief and the prevention of tilidine addiction through naloxone-induced antagonisms at the morphine receptor. A fixed combination of buprenorphine and naloxone was introduced in 1991 in New Zealand (Temgesic® Nx, Reckitt & Colman) for the treatment of pain.

WO 2009/023672 A2 relates to an abuse resistant oral pharmaceutical composition, comprising: a barrier layer, comprising a first polymer; and a diffusion layer, comprising a second polymer, substantially covering the barrier layer, wherein the diffusion layer is bonded to the barrier layer and comprises a drug that is substantially homogeneously distributed within the second polymer and diffuses from the diffusion layer within the gastrointestinal (GI) tract, wherein the pharmaceutical composition may optionally comprise an expansion layer comprising an expandable polymer and wherein the barrier layer substantially covers the expansion layer.

There exists a need in the art for a dosage form containing a drug susceptible to abuse that is resistant to parenteral and/or nasal abuse. In the case of opioid analgesics, there exists a need for a tamper resistant formulation that does not solely rely upon the inclusion of an antagonist in the formulation to deter parenteral and/or nasal abuse.

### SUMMARY OF THE INVENTION

The invention is defined by the appended claims. It is an object of certain embodiments of the present invention to provide an immediate release solid oral dosage form comprising an active agent (e.g., an opioid analgesic) which is tamper resistant.

It is an object of certain embodiments of the present invention to provide an immediate release solid oral dosage form comprising an active agent (e.g., an opioid analgesic) which is subject to less parenteral abuse than other dosage forms.

It is an object of certain embodiments of the present invention to provide an immediate release solid oral dosage form comprising an active agent (e.g., an opioid analgesic) which is subject to less intranasal abuse than other dosage forms.

It is a further object of certain embodiments of the present invention to provide an immediate release solid oral dosage form comprising an active agent (e.g., an opioid analgesic) which is subject to less diversion than other dosage forms.

It is a further object of certain embodiments of the present invention to treat a disease or condition (e.g., pain) in human patients by administering an immediate release solid oral dosage form as disclosed herein to a patient in need thereof.

It is a further object of certain embodiments of the present invention to provide an immediate release solid oral dosage form comprising an opioid analgesic for use in a method of treating pain in human patients with reduced abuse potential.

It is a further object of certain embodiments of the present invention to provide a method of manufacturing an immediate release solid oral dosage form of an active agent (e.g., an opioid analgesic) as disclosed herein.

It is a further object of certain embodiments of the present invention to provide a use of a medicament (e.g., an opioid analgesic) in the manufacture of a tamper-resistant dosage form as disclosed herein for the treatment of a disease state (e.g., pain).

The above objects of the present invention and others can be achieved by the present invention, which in certain embodiments is directed to an immediate release solid oral dosage form comprising a plurality of particles, each particle comprising (i) a core comprising a gelling agent; (ii) a barrier layer surrounding the core; and (iii) an active layer comprising a drug susceptible to abuse surrounding the barrier layer.

In certain embodiments, the plurality of particles are contained within a pharmaceutically acceptable capsule. In certain other embodiments, the plurality of particles are compressed into a tablet. In certain embodiments, the capsule or tablet further contains a pharmaceutically acceptable diluent.

In certain embodiments, the present invention is directed to a unit dose of an immediate release solid oral dosage form, said unit dose comprising from about 2 to about 75 particles, each particle comprising (i) a core comprising a gelling agent in the form of a compressed tablet; (ii) a barrier layer encompassing the core; and (iii) an active layer comprising a drug susceptible to abuse surrounding the core; wherein the dosage form releases at least 85% by weight of the drug within 45 minutes as measured by in-vitro dissolution in a USP Apparatus 1 (basket) at 100 rpm in 900 ml simulated gastric fluid without enzymes (SGF) at 37° C; wherein the viscosity of the dosage form mixed with from 0.5 to 10 ml of an aqueous liquid is at least 10 mPa s (10 cP); wherein the barrier layer comprises hydroxypropylmethylcellulose, polyvinyl alcohol, povidone or a mixture thereof, and wherein the barrier layer is applied to the core in an amount to provide a weight gain from 1 % (w/w) to 10 % (w/w).In certain embodiments, the unit dose is contained within a pharmaceutically acceptable capsule. In certain other embodiments, the unit dose is a tablet formed from compression of the particles. In certain embodiments, the unit dose further comprises a pharmaceutically acceptable diluent.

The solid oral dosage form disclosed herein releases at least about 85% by weight, or at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

In certain embodiments, the solid oral dosage form disclosed herein releases at least about 90% by weight, or at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

In other embodiments, the viscosity resulting from mixing a unit dose of the dosage form with from about 0.5 to about 10 ml of an aqueous liquid prevents the drug from being absorbed, or reduces the ability of the drug to be absorbed, by parenteral or nasal administration.

A process for preparing an immediate release solid oral dosage form comprising a plurality of particles as disclosed herein, comprises (i) preparing a plurality of cores, each core comprising a gelling agent; (ii) applying a barrier layer so that it surrounds each core; and (iii) applying an active layer comprising a drug susceptible to abuse so that it surrounds the barrier layer on each core.

In certain embodiments, the process may further comprise containing the particles within a pharmaceutically acceptable capsule to form a unit dose. In certain other embodiments, the process may further comprise compressing the particles into a tablet form to form a unit dose, comprising from about 2 to about 75 particles.

The process may be directed to the preparation of a dosage form as disclosed herein that releases at least about 85% by weight, or at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

The process may be directed to the preparation of a solid oral dosage form as disclosed herein that releases at least about 90% by weight, or at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

The process may be directed to the preparation of a dosage form as disclosed herein wherein the viscosity resulting from mixing a unit dose of the dosage form (crushed or uncrushed) with from about 0.5 to about 10 ml of an aqueous liquid prevents the drug from being absorbed, or reduces the ability of the drug to be absorbed, by parenteral or nasal administration.

In certain embodiments, the present invention is directed to an immediate release dosage form as disclosed herein for use in a method of treating a disease or condition (e.g., pain, diarrhea or constipation) comprising administering the dosage form to a patient in need thereof.

In describing the present invention, the following terms are to be used as indicated below. As used herein, the singular forms "a," "an," and "the" include plural references unless the context clearly indicates otherwise. Thus, for example, reference to "an active agent" includes a single active agent as well as a mixture of two or more different active agents, and reference to a "gelling agent" includes a single gelling agent as well as a mixture of two or more different gelling agents.

As used herein, the terms "active agent," "active ingredient," "pharmaceutical agent," and "drug" refer to any material that is intended to produce a therapeutic, prophylactic, or other intended effect, whether or not approved by a government agency for that purpose. These terms with respect to specific agents include all pharmaceutically active forms of the agent, including the free base form of the agent, and all pharmaceutically acceptable salts, complexes, stereoisomers, crystalline forms, cocrystals, ether, esters, hydrates, solvates, and mixtures thereof, where the form is pharmaceutically active.

As used herein, the terms "therapeutically effective" refers to the amount of drug or the rate of drug administration needed to produce a desired therapeutic result.

As used herein, the terms "prophylactically effective" refers to the amount of drug or the rate of drug administration needed to produce a desired prophylactic result.

As used herein, the term "stereoisomers" is a general term for all isomers of individual molecules that differ only in the orientation of their atoms in space. It includes enantiomers and isomers of compounds with one or more chiral centers that are not mirror images of one another (diastereomers).

The term "enantiomer" or "enantiomeric" refers to a molecule that is non-superimposable on its mirror image and hence optically active wherein the enantiomer rotates the plane of polarized light in one direction by a certain degree, and its mirror image rotates the plane of polarized light by the same degree but in the opposite direction.

The term "chiral center" refers to a carbon atom to which four different groups are attached.

The term "racemic" refers to a mixture of enantiomers.

The term "resolution" refers to the separation or concentration or depletion of one of the two enantiomeric forms of a molecule.

The term "patient" means a subject, particularly a human, who has presented a clinical manifestation of a particular symptom or symptoms suggesting the need for treatment, who is treated preventatively or prophylactically for a condition, or who has been diagnosed with a condition to be treated. The term "subject" is inclusive of the definition of the term "patient" and does not exclude individuals who are entirely normal in all respects or with respect to a particular condition.

"Pharmaceutically acceptable salts" include e.g. inorganic acid salts such as hydrochloride, hydrobromide, sulfate, phosphate; organic acid salts such as formate, acetate, trifluoroacetate, maleate, tartrate; sulfonates such as methanesulfonate, benzenesulfonate, p-toluenesulfonate; amino acid salts such as arginate, asparaginate, glutamate; metal salts such as sodium salt, potassium salt, cesium salt; alkaline earth metals such as calcium salt, magnesium salt; and organic amine salts such as triethylamine salt, pyridine salt, picoline salt, ethanolamine salt, triethanolamine salt, discyclohexylamine salt, N,N'-dibenzylethylenediamine salt.

The term "ppm" as used herein means "parts per million". Regarding 14-hydroxycodeinone, "ppm" means parts per million of 14-hydroxycodeinone in a particular sample product. The 14-hydroxycodeinone level can be determined by any method known in the art, preferably by HPLC analysis using UV detection.

### BRIEF DESCRIPTION OF THE DRAWINGS

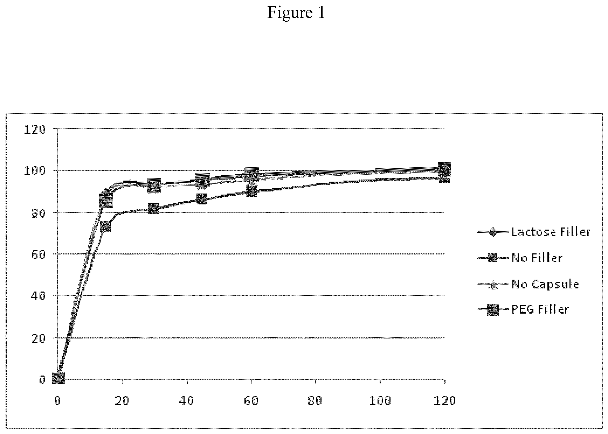

Figure 1 is a graphical depiction of the dissolution results of Example 6.

### DETAILED DESCRIPTION

Gelling agents have been contemplated for use in pharmaceutical formulations in order to deter the abuse of dosage forms containing a drug susceptible to abuse (e.g., an opioid analgesic). One form of abuse is the crushing of a controlled release dosage form in order to liberate the drug contained therein for illicit use such as parenteral administration or through absorption across a mucosal surface. When a dosage form having a gelling agent is crushed and then mixed with a solution, a viscosity is obtained which may inhibit the drug from being drawn into a needle, thereby hindering parenteral abuse. Similarly, when the crushed dosage form is applied to a mucosal surface (e.g., the nasal cavity) the composition will gel upon contact with mucosal moisture, thereby inhibiting absorption.

Controlled release dosage forms of drugs of abuse have received considerable attention in an attempt to develop tamper-resistant technologies as the crushing of the dosage form may liberate an amount of active agent normally intended for prolonged release (e.g., 12 to 24 hours)

Immediate release dosage forms are also the subject of abuse and present public safety issues when administered by other than the intended route. One problem to overcome in incorporating a gelling agent into an immediate release dosage form is controlled release characteristics that such an agent may impart to a dosage form when included in sufficient amounts to inhibit tampering.

In certain situations, an immediate release or controlled release dosage form can be abused without crushing, e.g., by contacting the intact dosage form with a liquid to dissolve the active agent contained therein. This can be a particular issue with intact dosage forms that are in particulate form, given the larger surface area and increased dissolution of such dosage forms.

Both controlled release and immediate release multiparticulate formulations may have formulation and pharmacokinetic issues such as (i) difficulty in manufacture, (ii) dose to dose variability in active agent, (iii) pharmacokinetic variability, (iv) variability due to administration with food, and (v) patient-to-patient variability which may be addressed by the present invention.

Immediate release and controlled release dosage forms play a vital role in the management of both acute and chronic conditions (e.g., pain management with opioid analgesics). Therefore, it is important to provide a tamper-resistant dosage form of a drug susceptible to abuse that may be utilized for either controlled or immediate release dosage forms to obtain a viable product that can provide effective plasma levels to a patient according to an intended release profile.

The present invention is directed to an oral dosage form comprising from 2 to 75 particles, each particle comprising:
(i) a core comprising a gelling agent in the form of a compressed tablet;
(ii) a barrier layer surrounding the core; and
(iii) an active layer comprising a drug susceptible to abuse encompassing the barrier layer;
wherein the dosage form releases at least 85% by weight of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid is at least 10 mPa s .
wherein the barrier layer comprises hydroxypropylmethylcelluclose, polyvinyl alcohol, povidone or a mixture therefore, and
wherein the barrier layer is applied to the core in an amount to provide a weight gain from 1 % (w/w) to 10 % (w/w).

In alternative embodiments, each particle of the immediate release dosage form may further comprise:
(iv) a second barrier layer encompassing the active layer; and
(v) a second active layer comprising a non-orally bioavailable opioid antagonist encompassing the second barrier layer.

The material and/or amount of material utilized in the barrier layer preferably will not substantially interfere with the release profile of the active agent from the dosage form. A material for the barrier layer can be, e.g., an acrylic polymer, a cellulosic polymer or a vinyl polymer. The barrier layers of the present invention include hydroxypropylmethylcellulose, polyvinyl alcohol, povidone or a mixture thereof.

In other embodiments, the plurality of particles are contained within a pharmaceutically acceptable capsule, preferably with the co-containment of an inert diluent. The diluent can be selected from saccharides (e.g., sucrose, dextrose, lactose, fructose, mannitol, and mixtures thereof), polyethylene glycols and cellulosic materials (e.g., microcrystalline cellulose).

The use of a barrier layer, co-containment with a diluent in a capsule, and selection of the number of particles in a unit dose, may all contribute to reducing agglomeration of the particles upon introduction to a dissolution medium either in vitro or in vivo (e.g., upon oral administration to a human subject) in order to maintain the immediate release profile of the dosage form.

In certain embodiments, the viscosity after mixing a dosage form (crushed or intact) with from about 0.5 to about 10 ml of an aqueous liquid is from about 10 cP to about 100 Cp; from about 25 cP to about 75 Cp; at least about 20 cP; at least about 40 cP or at least about 60 cP. In other embodiments, the viscosity after mixing a dosage form (crushed or intact) with from about 0.5 to about 10 ml of an aqueous liquid is at least about 10 cP, at least about 25 cP, at least about 75 Cp; at least about 100 cP; at least about 150 cP. One cP converts to one mPa s as the SI unit used in the claims.

In certain embodiments, the weight amount of gelling agent contained in the dosage form of the present invention is not more than the weight amount of drug. In other embodiments, the weight amount of gelling agent contained in the immediate release dosage forms of the present invention is less than the weight amount of drug. In further embodiments, the weight amount of gelling agent contained in the immediate release dosage forms of the present invention is more than the weight amount of drug.

In certain embodiments, the dosage forms of the present invention contain a weight ratio of gelling agent to drug from about 5:1 to about 1:5; from about 3:1 to about 1:3; from about 1:1 to about 1:1.5; from about 1.5:1 to about 1:1; about 1:1.25; or about 1.25:1.

The gelling agent utilized in the dosage forms of the present invention can be selected from sugars, sugar derived alcohols (e.g., mannitol, sorbitol), starch and starch derivatives, cellulose derivatives (e.g., microcrystalline cellulose, sodium caboxymethyl cellulose, methylcellulose, ethyl cellulose, hydroxyethyl cellulose, hydroxypropyl cellulose, and hydroxypropyl methylcellulose), attapulgites, bentonites, dextrins, alginates, carrageenan, gum tragacanth, gum acacia, guar gum, xanthan gum, pectin, gelatin, kaolin, lecithin, magnesium aluminum silicate, carbomers, carbopols, polyvinylpyrrolidone, polyethylene glycol, polyethylene oxide, polyvinyl alcohol, silicon dioxide, surfactants, mixed surfactant/wetting agent systems, emulsifiers, other polymeric materials, and mixtures thereof. In certain embodiments, the gelling agent is xanthan gum. In other embodiments, the gelling agent is pectin. The pectin or pectic substances include purified or isolated pectates and crude natural pectin from sources such as apple, citrus or sugar beet residues which have been subjected, when necessary, to esterification or de-esterification (e.g., by alkali or enzymes). The pectins may also be derived from citrus fruits such as lime, lemon, grapefruit, and orange. In preferred embodiments, the gelling agent is selected from the group consisting of polyethylene oxide, hydroxypropylcellulose, hydroxyethylcellulose, hydroxypropylmethylcellulose, and mixtures thereof.

A unit dose of a dosage form of the present invention includes from about 2 to about 75 particles; from about 10 to about 50 particles; from about 15 to about 25 particles; or from about 10 to about 50 particles. In certain embodiments of the present invention, each unit dose in a batch contains the same amount of particles. Such embodiments may be preferable to typical multiparticulate dosage forms which may contain a greater number of particles and may not uniformly contain the same amount of particulates in each unit dose. Further, typical multiparticulate dosage forms may not have content uniformity for each individual particle.

In certain embodiments, each particle contains a sub-therapeutic amount of active agent but collectively in a unit dosage form contain a therapeutic amount of the active agent.

Certain embodiments of the present invention may address formulation and pharmacokinetic issues such as (i) difficulty in manufacture, (ii) dose to dose variability in active agent, (iii) pharmacokinetic variability, (iv) variability due to administration with food, and (v) patient-to-patient variability.

The particles of the present invention may have a mean diameter from about 0.1 mm to about 10 mm; from about 0.5 mm to about 8 mm; from about 1 mm to about 6 mm; or from about 2 mm to about 4 mm.

The core of the particles of the formulation is in the form of a compressed tablet.

The dosage forms of the present invention may comprise without limitation, from about 25% to about 99% core by weight; from about 50% to about 95% core by weight; or from about 65% to about 85% core by weight.

The barrier layer is applied to the core in an amount to provide a weight gain from about 1%(w/w) to about 10%(w/w); or from about 4%(w/w) to about 7%(w/w).

The immediate release dosage form of the present invention releases at least about 85% by weight, at least about 90% by weight, or at least about 95% by weight of the drug within 45 minutes as measured by in-vitro dissolution in a USP Apparatus 1 (basket) at 100 rpm in 900 ml simulated gastric fluid without enzymes (SGF) at 37° C.

In certain embodiments, the immediate release dosage form of the present invention releases at least about 90% by weight, at least about 95% by weight, or at least about 98% by weight of the drug within 60 minutes as measured by in-vitro dissolution in a USP Apparatus 1 (basket) at 100 rpm in 900 ml simulated gastric fluid without enzymes (SGF) at 37° C.

### Additional Excipients

The compressed cores of the present invention can include additional excipients in order to, e.g., aid manufacturing, provide additional tamper resistance, modify the release rate, or provide alcohol resistance.

The additional excipient may be at least one excipient selected from the group consisting of bulking agents, plasticizers, stabilizers, diluents, lubricants, binders, granulating aids, colorants, flavorants, and glidants.

In certain embodiments, the dosage form includes a polymer that can modify the release rate of the active agent contained therein. Examples of polymers that can be utilized to modify the release of the active agent include pharmaceutically acceptable cellulosic polymers, including but not limited to cellulose esters, cellulose diesters, cellulose triesters, cellulose ethers, cellulose ester-ethers, cellulose acylates, cellulose diacylates, cellulose triacylates, cellulose acetates, cellulose diacetates, cellulose triacetates, cellulose acetate propionates, cellulose acetate butyrates and mixtures thereof. Preferably, the cellulosic polymer is an alkyl cellulosic polymer such as methylcellulose or ethylcellulose.

In other embodiments of the present invention, the release modifying polymer is a pharmaceutically acceptable acrylic polymer selected without limitation from from acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid) (anhydride), methyl methacrylate, polymethacrylate, poly(methyl methacrylate), poly(methyl methacrylate) copolymer, polyacrylamide, aminoalkyl methacrylate copolymer, poly(methacrylic acid anhydride), glycidyl methacrylate copolymers, and mixtures of any of the foregoing.

Preferably, the acrylic polymer is a neutral acrylic polymer (e.g., Eudragit NE 30 D®, Eudragit NE 40 D® or Eudragit NM 30 D®), which can also provide crush-resistant characteristics to the dosage form.

Individual compressed cores can also include a film coating or barrier coating to enhance cosmetic appearance, reduce tackiness and/or provide stability. Examples of materials to be utilized as a film or barrier coat include hydroxypropylmethylcellulose, polyvinyl alcohol, lactose or a mixture thereof. The inventive dosage forms include a barrier coat that is (i) a coating directly coated onto a compressed core, but can also comprise a film/barrier coat that is (ii) an outer coating directly coated onto a final layered compressed core, or (iii) an intermediate layer between any components of the dosage form.

### Alcohol Resistance

The gelling agent and the optional release modifying agent can be selected in order to inhibit dose dumping of the active agent in the presence of alcohol. This characteristic is to prevent the dosage form from releasing the active agent at a rate faster than intended when alcohol is imbibed during residence of the dosage form in the gastrointestinal tract. Certain hydrophilic polymers (e.g., polyethylene oxide or methylcellulose) are suitable gelling agents that can provide alcohol resistance to the dosage form.

In certain embodiments, the amount of active agent (e.g. an opioid analgesic) released at 1 hour in 900 mL 0.1 N HCl (pH 1.5) with 40% EtOH using USP Apparatus II at 50 rpm is not more than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is less than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 25%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 10%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is not more than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is less than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 25%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 10%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is not more than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is less than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 25%(w/w) of the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 10%(w/w) of the amount of active agent released at 4 hour in 900 mL 0.1 N HCl (pH 1.5) with 0% EtOH using USP Apparatus II at 50 rpm.

### Tamper Resistance

In certain embodiments, the solid oral dosage form of the present invention demonstrates the tamper-resistant characteristic of not breaking or shattering when force is applied to it (by, for example, striking it with a hammer). Instead, the solid oral dosage form flattens without breaking or shattering. This characteristic makes it more difficult for the solid oral dosage form to be abused, by snorting the powder of a shattered tablet, chewing a tablet, or injecting a solution prepared from a shattered tablet. The inclusion of polyethylene oxide can provide tamper-resistant properties. The addition of neutral acrylic polymer also provides these properties.

In certain embodiments, the oral solid dosage form can be flattened without breaking, wherein the thickness of the dosage form after flattening corresponds to no more than about 60%, no more than about 50%, no more than about 40%, no more than about 30%, or no more than about 20% of the thickness of the dosage form before flattening.

In certain embodiments, the amount of active agent (e.g. an opioid analgesic) released at 0.5 hour from a flattened dosage form deviates no more than about 20%, no more than about 15%, or no more than about 10% points from a non-flattened dosage form as measured by an in-vitro dissolution in a USP Apparatus 1 (basket) at 100 rpm in 900 ml simulated gastric fluid without enzymes (SGF) at 37° C.

### Active Agents

In certain embodiments, the active agent used in the solid oral dosage form of the present invention is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, anti-pyretics, anti-inflammatory agents, androgens, local and general anesthetics, anti-addictive agents, anti-androgens, anti-arrhythmic agents, antiasthmatic agents, anti-cholinergic agents, anti-cholinesterase agents, anti-coagulants, anti-diabetic agents, anti-diarrheal agents, anti-diuretic, anti-emetic agents, prokinetic agents, anti-epileptic agents, anti-estrogens, anti-fungal agents, antihypertensive agents, anti-microbial agents, anti-migraine agents, anti-muscarinic agents, anti-neoplastic agents, anti-parasitic agents, anti-parkinson's agents, antiplatelet agents, anti-progestins, anti-schizophrenia agents, anti-thyroid agents, anti-tussives, anti-viral agents, atypical anti-depressants, azaspirodecanediones, barbiturates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof.

In certain embodiments, the active agent is an opioid agonist. In such embodiments, the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof. In certain embodiments, the opioid agonist is selected from the group consisting of codeine, fentanyl, hydromorphone, hydrocodone, oxycodone, dihydrocodeine, dihydromorphine, morphine, tramadol, oxymorphone, pharmaceutically acceptable salts thereof, and mixture thereof.

In certain embodiments, the opioid agonist is oxycodone or a pharmaceutically acceptable salt thereof in an amount, e.g., of about 2.5 mg, 5 mg, 7.5 mg or 10 mg.

In certain embodiments of the present invention, wherein the active agent is oxycodone hydrochloride, oxycodone hydrochloride is used having a 14-hydroxycodeinone level of less than about 25 ppm, less than about 15 ppm, less than about 10 ppm, less than about 5 ppm, less than about 2 ppm, less than about 1 ppm, less than about 0.5 ppm or less than about 0.25 ppm.

WO 2005/097801 A1, U.S. Pat. No. 7,129,248 B2 and US 2006/0173029 A1, describe a process for preparing oxycodone hydrochloride having low levels of 14-hydroxycodeinone.

In certain embodiments, the oral solid dosage form of the present invention comprises an active agent that is an opioid antagonist (with or without an opioid agonist). In such embodiments, the opioid antagonist is selected from the group consisting of amiphenazole, naltrexone, methylnaltrexone, naloxone, nalbuphine, nalorphine, nalorphine dinicotinate, nalmefene, nadide, levallorphan, cyclozocine, pharmaceutically acceptable salts thereof and mixtures thereof.

In certain embodiments, the solid oral dosage form of the present invention comprises an active agent that is a non-opioid analgesic. In such embodiments, the non-opioid analgesic is a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.

In other embodiments, the present invention is directed to the dosage forms disclosed herein utilizing active agents such as benzodiazepines, barbiturates or amphetamines, their antagonists, or combinations thereof.

Benzodiazepines to be used in the present invention may be selected from alprazolam, bromazepam, chlordiazepoxide, clorazepate, diazepam, estazolam, flurazepam, halazepam, ketazolam, lorazepam, nitrazepam, oxazepam, prazepam, quazepam, temazepam, triazolam, and pharmaceutically acceptable salts, hydrates, and solvates and mixtures thereof. Benzodiazepine antagonists that can be used in the present invention include, but are not limited to, flumazenil and pharmaceutically acceptable salts, hydrates, and solvates.

Barbiturates to be used in the present invention include, but are not limited to, amobarbital, aprobarbotal, butabarbital, butalbital, methohexital, mephobarbital, metharbital, pentobarbital, phenobarbital, secobarbital and pharmaceutically acceptable salts, hydrates, and solvates mixtures thereof. Barbiturate antagonists that can be used in the present invention include, but are not limited to, amphetamines and pharmaceutically acceptable salts, hydrates, and solvates.

Stimulants to be used in the present invention include, but are not limited to, amphetamines, such as amphetamine, dextroamphetamine resin complex, dextroamphetamine, methamphetamine, methylphenidate and pharmaceutically acceptable salts, hydrates, and solvates and mixtures thereof. Stimulant antagonists that can be used in the present invention include, but are not limited to, benzodiazepines, and pharmaceutically acceptable salts, hydrates, and solvates as described herein.

### Methods of Manufacture

A process for preparing a dosage form as disclosed herein comprises:
(i) preparing a plurality of cores, each core comprising a gelling agent;
(ii) applying a barrier layer to surround each core; and
(iii) applying an active layer comprising a drug susceptible to abuse so that it surrounds the barrier layer on each core;
wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The process comprises:
(i) compressing (e.g., into a tablet) a plurality of cores comprising a gelling agent;
(ii) applying a barrier layer surrounding each core; and
(iii) applying an active layer comprising a drug susceptible to abuse so that it surrounds the barrier layer;
wherein the dosage form releases at least about 85% by weight of the drug within 45 minutes as measured by in-vitro dissolution in a USP Apparatus 1 (basket) at 100 rpm in 900 ml simulated gastric fluid without enzymes (SGF) at 37° C;
wherein the viscosity resulting from mixing a crushed unit dose of the dosage form with from about 0.5 to about 10 ml of an aqueous liquid prevents the drug from being absorbed, or reduces the ability of the drug to be absorbed, by parenteral or nasal administration.

The process comprises preparing an immediate release oral dosage form comprising from about 2 to about 75 particles prepared according to the invention.

The particles can be further processed into a unit dosage form, e.g., by containment in a capsule; containment in a sachet or paper; or compression into a tablet.

When the particles are contained in a capsule, certain embodiments include the co-containment of a diluent in the capsule along with the active agent particles. The incorporation of the diluent may serve to reduce agglomeration of the particles which can facilitate the dosage form maintaining the desired immediate release profile.

The diluent can be mixed with the particles and the mixture added to the capsule. Alternatively, the diluent can be added to the capsule before or after adding the active agent particles into the capsule.

In a preferred embodiment, the active agent particles are contained in the capsule and back-filled with the diluent.

The diluent can be as disclosed above and is preferably lactose or polyethylene glycol.

### Treatment

The present invention is further directed to any of the solid oral dosage forms described herein for use in a method of treating a disease or condition comprising administering the solid oral dosage form to a patient in need thereof. In certain embodiments, the patient is treated for pain, diarrhea, or constipation.

The solid oral dosage forms described herein may be for use in a method of treatment comprising administering the solid oral dosage form described herein in combination with another pharmaceutical composition. In certain embodiments, the other pharmaceutical composition is administered to treat the same condition or disease. In other embodiments, the other pharmaceutical composition is administered to treat a different condition or disease.

In certain embodiments, the dosage forms may be for use in a method of treatment further comprising monitoring the patient for how the patient metabolizes the active agent, or how the patient responds to the active agent. In certain embodiments, the dosage forms may be for use in a method of treatment further comprising altering the dose of the solid oral dosage form in response to said monitoring. In certain embodiments, the dosage forms may be for use in a method of treatment wherein certain baseline measurements are taken from the patient prior to administering the oral solid dosage form to the patient.

The following examples are set forth to assist in understanding the invention

### Examples

### Example 1

### Preparation of Core Formulation

Cores comprising a gelling agent were prepared in accordance with Table 1:

**TABLE 1**

| Ingredient | mg / unit | Actual Amt Used (g) |
|---|---|---|
| Polyethylene Oxide (PEO 303) | 3.96 | 1980 |
| Magnesium Stearate | 0.04 | 20 |
| Total | 4.0 | 2000 |

The ingredients were processed according to the following procedure:
1. The polyethylene oxide was added to a polyethylene bag.
2. The magnesium stearate was added and bag blended with the polyethylene oxide for a sufficient time to obtain a substantially uniform blend.
3. The blend was compressed with a Kilian/IMA tablet press with 2 mm multi-tipped tooling with the following parameters:
   Target Tablet Weight: 4 mg
   Fill Depth/Setting: 3.00 - 3.05 mm
   Main Compression Force Target: 4 Kn
   Actual Compression Force: 3.5 - 4.5 Kn
   Actual Tablet Weights: 4.00 - 4.20 mg
   Actual Tablet Thickness: approximately 1.5 mm

### Example 2

### Preparation of Barrier Layered Core Formulation

The cores of Example 1 were coated in accordance with Table 2:

**TABLE 2**

| Ingredient | mg / unit | Actual Amt Used (g) |
|---|---|---|
| Example 1 Cores | 4.00 | |
| Opadry | 0.24 | |
| DI Water* | | |
| **Total** | 4.24 | |

| | | |
|---|---|---|
| *DI Water is removed during processing and is not included in final weight | | |

The ingredients were processed according to the following procedure:
1. The DI water was added to a mixer and a vortex was created.
2. The Opadry was added to the DI water and mixed for about 1 hour.
3. The Vector VFC-3 Fluid Bed Processor, 4 liter chamber fitted with a Wurster Column was used to layer the solutions onto the substrate.
4. The processing parameters were as follows: The inlet temperature set point was about 65° to about 70° C; the product temperature was approximately 45° to about 50° C; the exhaust temperature was approximately 50° to about 55° C; the solution spray rate was approximately 4 grams per minute and the process air volume was approximately 70 cfm.
5. The Opadry solution was coated onto the cores of Example 1 to a weight gain of about 6%.

### Example 3

### Preparation of Active Layered Core Formulation

The barrier layered cores of Example 2 were coated in accordance with Table 3:

**Table 3**

| Ingredient | mg / barrier layered core | 20 barrier layered cores / unit | Actual Amt Used (g) |
|---|---|---|---|
| Barrier Layered Cores of Example 2 | 4.24 | 88.4 | 442 |
| Naltrexone HCl | 0.25 | 5 | 25 |
| Opadry | 0.318 | 6.36 | 31.8 |
| DI Water* | n/a* | n/a* | n/a* |
| **Total** | 4.988 | 99.76 | 498.8 |

| | | | |
|---|---|---|---|
| *DI Water is removed during processing and is not included in final weight | | | |

1. The DI water was added to a mixer and a vortex was created.
2. The Naltrexone HCl was added to the DI water and mixed until fully dispersed.
3. The Opadry was added to the Naltrexone HCl solution and mixed for about 1 hour.
4. The Vector VFC-3 Fluid Bed Processor, 4 liter chamber fitted with a Wurster Column was used to layer the solutions onto the sub-coated substrate.
5. The processing parameters were as follows: The product temperature set point was about 47° C; the product temperature was approximately 45° to about 50° C; the exhaust temperature was approximately 50° to about 55° C; the solution spray rate was approximately 4 grams per minute and the process air volume was approximately 80 cfm.
6. The Opadry/Naltrexone solution was coated onto the barrier layered cores of Example 2 to a weight gain of about 12.9%.

### Example 4

### Preparation of Seal Layered Core Formulation

The active layered cores of Example 3 were coated in accordance with Table 4:

**Table 4**

| Ingredient | mg / active layered core | 20 active layered cores / unit | Actual Amt Used (g) |
|---|---|---|---|
| Active Layered Cores of Ex. 3 | 5.066 | 101.32 | 450 |
| Opadry | 0.304 | 6.08 | 27.0 |
| DI Water* | n/a* | n/a* | n/a* |
| **Total** | 5.370 | 107.4 | 477.0 |

| | | | |
|---|---|---|---|
| *DI Water is removed during processing and is not included in final weight | | | |

1. The DI water was added to a mixer and a vortex was created.
2. The Opadry was added to the DI Water and mixed for about 1 hour.
3. The Vector VFC-3 Fluid Bed Processor, 4 liter chamber fitted with a Wurster Column was used to layer the solutions onto the sub-coated substrate.
4. The processing parameters were as follows: The product temperature set point was about 47° C; the product temperature was approximately 48°C; the exhaust temperature was approximately 45° to about 55° C; the solution spray rate was approximately 4 grams per minute and the process air volume was approximately 70 cfm.
5. The Opadry solution was coated onto the Active layered cores of Example 3 to a weight gain of 6.0%.

### Example 5

### Active Layered Core Formulation Dissolution (Total Amount)

5 mg naltrexone HCl multiple tablet formulations were prepared in accordance with the present invention. The formulations were tested in SGF for the mg amount released at specific time points.

Formulation A was encapsulated with a lactose filler.

Formulation B was encapsulated without a filler.

Formulation C was directly added to the dissolution medium without a capsule.

Formulation D was encapsulated with a polyethylene glycol filler.

The results are set forth in Table 5 below:

**Table 5**

| | **Sample wt (mg) (capsule included)** | **Vessel** | **15.0 min** | **30.0 min** | **45.0 min** | **60.0 min** | **120.0 min** | **720.0 min** |
|---|---|---|---|---|---|---|---|---|
| Formulation A-1 | 259.77 | A | 4.217 | 4.433 | 4.499 | 4.573 | 4.689 | 4.563 |
| Formulation A-1 | 222.45 | A | 4.316 | 4.523 | 4.628 | 4.717 | 4.874 | 4.937 |
| Formulation A-1 | 276.20 | A | 4.425 | 4.666 | 4.768 | 4.834 | 4.976 | 5.070 |
| **Mean (n=3)** | | | **4.32** | **4.54** | **4.63** | **4.71** | **4.85** | **4.86** |
| Formulation B -1 | 156.85 | A | 3.756 | 4.080 | 4.260 | 4.403 | 4.737 | 4.943 |
| Formulation B -1 | 160.37 | A | 3.593 | 4.059 | 4.272 | 4.475 | 4.836 | 5.055 |
| Formulation B -1 | 158.99 | A | 3.666 | 4.166 | 4.451 | 4.689 | 5.042 | 5.110 |
| **Mean(n=3)** | | | **3.67** | **4.10** | **4.33** | **4.52** | **4.87** | **5.04** |
| Formulation C -1 | 188.56 | B | 4.307 | 4.507 | 4.609 | 4.728 | 4.916 | 4.980 |
| Formulation C -1 | 186.30 | B | 4.376 | 4.542 | 4.626 | 4.708 | 4.870 | 4.913 |
| Formulation C -1 | 187.31 | B | 4.349 | 4.529 | 4.594 | 4.699 | 4.879 | 4.921 |
| **Mean** | | | **4.34** | **4.53** | **4.61** | **4.71** | **4.89** | **4.94** |
| Formulation D -1 | 402.02 | B | 4.021 | 4.478 | 4.628 | 4.733 | 4.885 | 4.907 |
| Formulation D -1 | 467.58 | B | 4.164 | 4.472 | 4.602 | * | * | 4.782 |
| Formulation D -1 | 425.72 | B | 4.374 | 4.669 | 4.824 | 4.879 | 4.974 | 4.993 |
| **Mean(n=3)** | | | **4.19** | **4.54** | **4.68** | **4.81** | **4.93** | **4.89** |
| ***Samples were not pulled for these time points for vessel #5 of Bath B.** | | | | | | | | |

### Example 6

### Active Layered Core Formulation Dissolution (% Released)

Formulations A-D were tested in SGF for the total amount of naltrexone HCl released over time. The results are set forth in Figure 1 and Table 6 below:

**Table 6**

| **Filler Type** | **0** | **15** | **30** | **45** | **60** | **120** |
|---|---|---|---|---|---|---|
| Formulation A | 0 | 89 | 93 | 95 | 97 | 100 |
| Formulation B | 0 | 73 | 81 | 86 | 90 | 97 |
| Formulation C | 0 | 88 | 92 | 93 | 95 | 99 |
| Formulation D | 0 | 86 | 93 | 96 | 98 | 101 |

### Example 7

### Syringability of Active Layered Core Formulation

A. Materials
Scintillation vial (hand shake for 1∼20 min)
1 cc Insulin syringe 28 ½ gauge needle

B. One active layered core of Example 3 was placed in 1 mL of water. The syringability was tested after specified time periods. The results are set forth in Table 7A below:

**Table 7A**

| Time | Syringability |
|---|---|
| 1∼5 min | slow to pull up |
| 10 min | slightly viscous, still syringable |
| 20 min | more viscous, still syringable |

C. 20 active layered cores of Example 3 were placed in 5 mL of water. The syringability was tested after specified time periods. The results are set forth in Table 7B below:

**Table 7B**

| Time | Syringability |
|---|---|
| 1 min | slow to draw up |
| 3 min | same as 1 min |
| 5 min | foamy, difficult to syringe |
| 10 min | very difficult to pull up |

### Example 8

### Syringability of Multiple Active Layered Core Formulations

Multiple tablet formulations prepared in accordance with the present invention were crushed, diluted with 2 ml of water and heated. Variations were as follows:

Formulation 8A was 1 dose (20 tablets) crushed between 2 tablespoons, diluted with 2 ml of water, and heated for 2 minutes (while stirring with a needle) with a butane lighter.

Formulation 8B was a double dose (40 tablets) crushed with a mortar and pestle, transferred to a spoon and heated for 2 minutes (while stirring with a needle) with a butane lighter.

Formulation 8C was a double dose crushed with a mortar and pestle, transferred to a spoon and heated for 2 minutes dry. 2 ml of water was then added and the mixture was stirred with a needle.

Formulations 8D to 8H were all performed using 40 tablets placed in 20 ml scintillation vials. 2 ml of water was added to each and then they each were shaken for 1, 3, 5, 12 & 15 minutes.

Formulation 8D was shaken by hand. Formulations 8E to 8G were mixed on a vortex. Formulation 8H was placed on a wrist action shaker. The liquid was removed using a 1 ml insulin syringe and then placed in a 25 ml volumetric flask and filled with SGF.

Results are set forth in Table 8 below:

**Table 8**

| Formulation | Volume drawn in ml | Assay results in mg of drug | % of theoretical | Appearance & observations |
|---|---|---|---|---|
| 8A | 0.8 | 1.7 | 34.2 | Difficult to crush using 2 spoons. Took 2 minutes to draw up. |
| 8B | 0.15 | 0.6 | 6.4 | Purple in color, much more viscous. Complete glob after 3 minutes. Unable to effectively draw up. |
| 8C | 0.7 | 2.6 | 25.8 | Thin, but not easily syringed. Cotton used after a few minutes. Took 6 minutes to draw up. |
| 8D | 2 | 8.3 | 82.8 | Liquid was grayish pink, easily drawn up. |
| 8E | 1.5 | 7.3 | 72.7 | Liquid was grayish pink, slight issue with tablets drawn to needle. |
| 8F | 1.35 | 6.5 | 65.3 | Liquid was grayish pink, more difficult to avoid tablets on tip of needle. |
| 8G | 1.2 | 5.2 | 52.3 | Liquid was grayish pink, tablets were blocking syringe, they were removed from liquid at top of vial. Difficulty drawing up. |
| 8H | 1.4 | 5.8 | 58.4 | Liquid was grayish pink. Similar to 8G. |

### Reference Example 9

### Preparation of Controlled Release Formulation (Prophetic)

The active layered cores of Example 3 are coated with a controlled release coating as follows:
1. An aqueous dispersion of a neutral copolymer based on ethyl acrylate and methyl methacrylate (Eudragit NE 40 D) is coated onto the subsrate with a Vector VFC-3 Fluid Bed Processor, 4 liter chamber fitted with a Wurster Column.
2. The processing parameters are as follows: The product temperature set point is set at ambient temperature; the product temperature is ambient temperature; the exhaust temperature is approximately 45° to about 55° C; the spray rate is approximately 4 grams per minute and the process air volume is approximately 70 cfm.
3. The Eudragit NE 40 D dispersion is coated onto the Active layered cores of Example 3 to a weight gain of 20%.

### Reference Example 10

### Preparation of Controlled Release Formulation (Prophetic)

The active layered cores of Example 3 are coated with a controlled release coating as follows:
1. An aqueous dispersion of a ethylcellulose (Surelease) is coated onto the subsrate with a Vector VFC-3 Fluid Bed Processor, 4 liter chamber fitted with a Wurster Column.
2. The processing parameters are as follows: The product temperature set point is set at ambient temperature; the product temperature is ambient temperature; the exhaust temperature is approximately 45° to about 55° C; the spray rate is approximately 4 grams per minute and the process air volume is approximately 70 cfm.
3. The Eudragit NE 40 D dispersion is coated onto the Active layered cores of Example 3 to a weight gain of 20%.

## Claims

1. An oral dosage form comprising from 2 to 75 particles, each particle comprising:
(i) a core comprising a gelling agent in the form of a compressed tablet;
(ii) a barrier layer encompassing the core; and
(iii) an active layer comprising a drug susceptible to abuse encompassing the barrier layer;
wherein the dosage form releases at least 85% by weight of the drug within 45 minutes as measured by in-vitro dissolution in a USP Apparatus 1 (basket) at 100 rpm in 900 ml simulated gastric fluid without enzymes (SGF) at 37° C;
wherein the viscosity of the dosage form mixed with from 0.5 to 10 ml of an aqueous liquid is at least 10 mPa s (10 cP),
wherein the barrier layer comprises hydroxypropylmethylcellulose, polyvinyl alcohol, povidone or a mixture thereof, and
wherein the barrier layer is applied to the core in an amount to provide a weight gain from 1 % (w/w) to 10 % (w/w).

2. The dosage form of claim 1, wherein the ratio of gelling agent to drug is from 5:1 to 1:5.

3. The oral dosage form of claim 1, wherein the gelling agent is selected from the group consisting of sugars, sugar derived alcohols, cellulose derivatives, gums, polymers, and mixtures thereof.

4. The oral dosage form of claim 1, wherein the gelling agent is selected from the group consisting of polyethylene oxide, hydroxypropylcellulose, hydroxyethylcellulose, hydroxypropylmethylcellulose, and mixtures thereof, and preferably is polyethylene oxide.

5. The dosage form of claim 1, wherein the from 2 to 75 particles comprise a therapeutically effective amount of the drug.

6. The dosage form of claim 1, wherein the drug is selected from the group consisting of an opioid agonist, a tranquilizer, a CNS depressant, a CNS stimulant, a sedative hypnotic, and mixtures thereof.

7. The dosage form of any of claims 1-6, wherein the drug is an opioid agonist, wherein the opioid agonist is preferably selected from the group consisting of codeine, morphine, oxycodone, oxymorphone, hydrocodone, hydromorphone, pharmaceutically acceptable salts thereof, and mixtures thereof.

8. The dosage form of claim 7, wherein the opioid agonist is oxycodone or a pharmaceutically acceptable salt thereof.

9. The dosage form of claim 8, comprising 5 mg oxycodone or a pharmaceutically acceptable salt thereof.

10. The dosage form of any of claims 1-9, comprising from 10 to 50 particles.

11. The dosage form of any of claims 1-10, comprising from 25% by weight to 99% by weight core, preferably from 50% by weight to 95% by weight core and more preferably from 65% by weight to 85% by weight core.

12. The dosage form of any of claims 1-11, wherein the barrier layer is applied to the core in an amount to provide a weight gain from 4 % (w/w) to 7 % (w/w).

13. The dosage form of any of claims 1-12, wherein the particles are contained within a pharmaceutically acceptable capsule.

14. The dosage form of claim 13, further comprising a diluent contained within the pharmaceutically acceptable capsule, wherein the diluent is preferably a saccharide.

15. The dosage form of any of claims 1-14 for use in the method of treating a disease or condition.

## Patentansprüche

1. Orale Darreichungsform, umfassend 2 bis 75 Teilchen, wobei jedes Partikel Folgendes umfasst:
(i) einen Kern, umfassend ein Geliermittel in Form einer komprimierten Tablette;
(ii) eine Barriereschicht, die den Kern umgibt; und
(iii) eine aktive Schicht, die einen für einen Missbrauch empfänglichen Arzneistoff umfasst, welche die Barriereschicht umgibt;
wobei die Darreichungsform mindestens 85 Gew.-% des Arzneistoffs innerhalb von 45 Minuten freisetzt, gemessen durch In-vitro-Auflösung in einem USP-Gerät 1 (Korb) bei 100 U/min in 900 ml künstlichem Magensaft ohne Enzyme (SGF) bei 37 °C;
wobei die Viskosität der Darreichungsform, gemischt mit 0,5 bis 10 ml einer wässrigen Flüssigkeit, mindestens 10 mPa·s (10 cP) beträgt,
wobei die Barriereschicht Hydroxypropylmethylcellulose, Polyvinylalkohol, Povidon oder ein Gemisch davon umfasst, und
wobei die Barriereschicht in einer Menge auf den Kern aufgebracht wird, um eine Gewichtszunahme von 1 % (Gew./Gew.) bis 10 % (Gew./Gew.) bereitzustellen.

2. Darreichungsform nach Anspruch 1, wobei das Verhältnis von Geliermittel zu Arzneistoff von 5:1 bis 1:5 beträgt.

3. Orale Darreichungsform nach Anspruch 1, wobei das Geliermittel aus der Gruppe bestehend aus Zuckern, von Zucker abgeleiteten Alkoholen, Cellulosederivaten, Gummis, Polymeren und Gemischen davon ausgewählt ist

4. Orale Darreichungsform nach Anspruch 1, wobei das Geliermittel aus der Gruppe bestehend aus Polyethylenoxid, Hydroxypropylcellulose, Hydroxyethylcellulose, Hydroxypropylmethylcellulose und Gemischen davon ausgewählt ist und vorzugsweise Polyethylenoxid ist.

5. Darreichungsform nach Anspruch 1, wobei die 2 bis 75 Teilchen eine therapeutisch wirksame Menge des Arzneistoffs umfassen.

6. Darreichungsform nach Anspruch 1, wobei der Arzneistoff aus der Gruppe bestehend aus einem Opioidagonisten, einem Beruhigungsmittel, einem auf das ZNS dämpfend wirkenden Mittel, einem ZNS-Stimulans, einem sedativen Hypnotikum und Gemischen davon ausgewählt ist.

7. Darreichungsform nach einem der Ansprüche 1-6, wobei der Arzneistoff ein Opioidagonist ist, wobei der Opioidagonist vorzugsweise aus der Gruppe, bestehend aus Codein, Morphin, Oxycodon, Oxymorphon, Hydrocodon, Hydromorphon, pharmazeutisch annehmbaren Salzen davon und Gemischen davon ausgewählt ist.

8. Darreichungsform nach Anspruch 7, wobei der Opioidagonist Oxycodon oder ein pharmazeutisch annehmbares Salz davon ist.

9. Darreichungsform nach Anspruch 8, umfassend 5 mg Oxycodon oder ein pharmazeutisch annehmbares Salz davon.

10. Darreichungsform nach einem der Ansprüche 1-9, umfassend 10 bis 50 Teilchen.

11. Darreichungsform nach einem der Ansprüche 1-10, umfassend 25 Gew.-% bis 99 Gew.-% Kern, vorzugsweise 50 Gew.-% bis 95 Gew.-% Kern und mehr bevorzugt 65 Gew.-% bis 85 Gew.-% Kern.

12. Darreichungsform nach einem der Ansprüche 1-11, wobei die Barriereschicht in einer Menge auf den Kern aufgebracht ist, um eine Gewichtszunahme von 4 % (Gew./Gew.) bis 7 % (Gew./Gew.) bereitzustellen.

13. Darreichungsform nach einem der Ansprüche 1-12, wobei die Teilchen in einer pharmazeutisch annehmbaren Kapsel enthalten sind.

14. Darreichungsform nach Anspruch 13, ferner umfassend ein Verdünnungsmittel, das in der pharmazeutisch annehmbaren Kapsel enthalten ist, wobei das Verdünnungsmittel vorzugsweise ein Saccharid ist.

15. Darreichungsform nach einem der Ansprüche 1-14 zur Verwendung in dem Verfahren zum Behandeln einer Krankheit oder eines Zustands.

## Revendications

1. Forme galénique orale comprenant de 2 à 75 particules, chaque particule comprenant :
(i) un noyau comprenant un agent de gélification sous la forme d'une tablette comprimée ;
(ii) une couche de barrière englobant le noyau ; et
(iii) une couche active comprenant un médicament pouvant créer une dépendance englobant la couche de barrière ;
dans laquelle la forme galénique libère au moins 85 % en poids du médicament dans les 45 min mesuré par dissolution in vitro dans un Appareil USP 1 (panier) à 100 tpm dans 900 ml de suc gastrique artificiel (SGF) en absence d'enzymes à 37 °C ;
dans laquelle la viscosité de la forme galénique mélangée avec de 0,5 à 10 ml d'un liquide aqueux est d'au moins 10 mPa/s (10 cP),
dans laquelle la couche barrière comprend de l'hydroxypropylméthylcellulose, l'alcool polyvinylique, le povidone ou un mélange de ceux-ci, et
dans laquelle la couche barrière est appliquée au noyau en une quantité pour fournir un gain en poids de 1 % (p/p) à 10 % (p/p).

2. Forme galénique selon la revendication 1, dans laquelle le rapport de l'agent de gélification sur le médicament est de 5:1 à 1:5.

3. Forme galénique orale selon la revendication 1, dans laquelle l'agent de gélification est choisi dans le groupe composé de sucres, d'alcools dérivés de sucre, des dérivés de cellulose, des gommes, des polymères et des mélanges de ceux-ci.

4. Forme galénique orale selon la revendication 1, dans laquelle l'agent de gélification est choisi dans le groupe composé de l'oxyde de polyéthylène, de l'hydroxypropylcellulose, de l'hydroxyéthylcellulose, de l'hydroxypropylméthylcellulose et des mélanges de ceux-ci, et de préférence est l'oxyde de polyéthylène.

5. Forme galénique selon la revendication 1, dans laquelle les 2 à 75 particules comprennent une quantité thérapeutiquement efficace du médicament.

6. Forme galénique selon la revendication 1, dans laquelle le médicament est choisi dans le groupe composé d'un agoniste d'opioïde, d'un tranquillisant, d'un dépresseur du SNC, d'un stimulant du SNC, d'un hypnotique sédatif, et des mélanges de ceux-ci.

7. Forme galénique selon l'une quelconque des revendications 1 à 6, dans laquelle le médicament est un agoniste d'opioïde, dans laquelle l'agoniste d'opioïde est de préférence choisi dans le groupe composé de la codéine, de la morphine, de l'oxycodone, de l'oxymorphone, de l'hydrocodone, de l'hydromorphone, des sels pharmaceutiquement acceptables de ceux-ci et des mélanges de ceux-ci.

8. Forme galénique selon la revendication 7, dans laquelle l'agoniste d'opioïde est l'oxycodone ou un sel pharmaceutiquement acceptable de celui-ci.

9. Forme galénique selon la revendication 8 comprenant 5 mg d'oxycodone ou d'un sel pharmaceutiquement acceptable de celui-ci.

10. Forme galénique selon l'une quelconque des revendications 1 à 9, comprenant de 10 à 50 particules.

11. Forme galénique selon l'une quelconque des revendications 1 à 10, comprenant de 25 % en poids à 99 % en poids de noyau, de préférence de 50 % en poids à 95 % en poids de noyau et idéalement de 65 % en poids à 85 % en poids de noyau.

12. Forme galénique selon l'une quelconque des revendications 1 à 11, dans laquelle la couche barrière est appliquée au noyau en une quantité pour fournir un gain en poids de 4 % (p/p) à 7 % (p/p).

13. Forme galénique selon l'une quelconque des revendications 1 à 12, dans laquelle les particules sont contenues à l'intérieur d'une capsule pharmaceutiquement acceptable.

14. Forme galénique selon la revendication 13, comprenant également un diluant contenu à l'intérieur de la capsule pharmaceutiquement acceptable, dans laquelle le diluant est de préférence un saccharide.

15. Forme galénique selon l'une quelconque des revendications 1 à 14, pour une utilisation dans le procédé de traitement d'une maladie ou d'un état.
